# EUROPEAN PATENT APPLICATION

(11) **EP 3 075 391 A1**
(43) Date of publication of application: **05.10.2016**
(21) Application number: 16161449.0
(22) Date of filing: 16.02.2009
(51) Int. Cl.: A61K 39/00, C07K 14/755, C12N 9/02, A61K 39/39

(54) **STRATEGIES TO PREVENT AND/OR TREAT IMMUNE RESPONSES TO SOLUBLE ALLOFACTORS**

(30) Priority: 14.02.2008 EP 08447010; 12.03.2008 US 35800 P
(62) Divisional of application: 09710097.8
(71) Applicant: Life Sciences Research Partners VZW, 3000 Leuven (BE); Katholieke Universiteit Leuven, 3000 Leuven (BE)
(72) Inventor: Saint-Remy, Jean-Marie, 1390 Grez-Doiceau (BE)
(74) Representative: IPLodge bvba

(57) **Abstract**

The invention relates to isolated immunogenic peptides comprising a T-cell epitope derived from a soluble allofactor and a C-(X)2-[CST] or [CST]-(X)2-C motif, which is immediately adjacent or separated from the T-cell epitope by at most 7 amino acids. The application further relates to the use of these peptides in preventing or suppressing an immune response to a soluble allofactor. The invention further relates to the use of these peptides in obtaining populations of soluble allofactor-specific regulatory T cells with cytotoxic properties.

## Description

### FIELD OF THE INVENTION

The present invention relates to immunogenic peptides and their use in preventing and/or suppressing immune responses to soluble (therapeutic) allofactor such as used in replacement therapy.

### BACKGROUND OF THE INVENTION

An increasing number of polypeptides or proteins and factors are used for administration in the setting of a large number of diseases. These include
- replacement therapy for coagulation defects or fibrinolytic defects, including factor VIII, factor IX and staphylokinase,
- hormones such as growth hormone or insulin,
- cytokines and growth factors, such as interferon-alpha, interferon-gamma, GM-CSF and G-CSF,
- antibodies for the modulation of immune responses, including anti-IgE antibodies in allergic diseases, anti-CD3 and anti-CD4 antibodies in graft rejection and a variety of autoimmune diseases, anti-CD20 antibodies in non-Hodgkin lymphomas,
- erythropoietin in renal insufficiency.

In many cases, administration of such polypeptides or proteins or factors elicits the production of a specific immune response. Antibodies produced towards these polypeptides and proteins or factors result in either the neutralisation of the therapeutic effect, an increase in clearance rate and diverse modes of hypersensitivity reactions, including serum sickness, anaphylactic reactions and cutaneous eruptions.

Antibodies elicited towards the therapeutic agent are produced by specific B lymphocytes, which are turned into effective antibody-forming cells by maturation and differentiation, which require both the presence of the antigen (i.e. the therapeutic agent) and the help provided by specific T cells. Thus, the polypeptide or protein is taken up by cells specialised in the presentation of antigens to the immune system, called antigen-presenting cells (APC). Such APC are located at sites at which the polypeptide or protein is administered: the spleen in case of intravenous administration, the skin for subcutaneous administration and regional lymph nodes for muscle administration.

APCs are broadly separated in two categories, i.e. professional and non-professional APC. Thus, dendritic cells and B cells (or B lymphocytes) are considered as professional APC because of their capacity to capture an antigen. Dendritic cells capture antigens by non-specific uptake followed by active processing and presentation at the cell surface of peptides derived from the antigen in combination with determinants of the major histocompatibility complex (MHC). B lymphocytes capture antigens by way of their specific surface receptor (B cell receptor, BCR) followed by processing and presentation in the context of MHC molecules. Non-professional APCs are mainly macrophages with relatively poor capacity to present antigen, somewhat compensated by their capacity to accumulate to sites of inflammation.

Primary immune responses are elicited by antigen uptake by dendritic cells or macrophages, whilst secondary responses are mainly dependent of specific B cells. This is due to the high capacity of dendritic cells to take up the antigen, compared to naïve B cells which are very poor at this activity. During a secondary immune response, however, when the maturation of BCR has already been obtained, B cells are by far the most efficient presenting cells.

As stated above, uptake of an antigen is followed by processing and presentation by MHC molecules. MHCs are divided in two categories, class I and class II, encoded by different gene loci. In man, three loci encode antigens of class I, called A, B and C, and three loci encode for class II antigens, called DP, DQ and DR.

The function of MHC antigens is to present peptides to T cells. It is classically considered that class I antigens present peptides mainly derived from cell endogenous antigens, while class II antigens present peptides generated by the processing of antigens from the outside. Distinct pathways of processing and presentation at cell surface have been described for class I as compared to class II antigen presentation.

The production of specific antibodies requires presentation of peptides into MHC class II determinants, which allows the recognition by and activation of T cells of the CD4+ subset. Upon recognition and activation, such CD4+ T cells produce a number of cytokines, which provide help to B cells for full maturation into antibody forming cells.

Any method that would abort immune responses to soluble therapeutic allofactors, both in the setting of primary responses (involving mainly dendritic cells) or ongoing responses (involving mainly B cells), would constitute a significant improvement in the types of treatment such as mentioned above.

### SUMMARY OF THE INVENTION

The present invention relates to the use of immunogenic peptides for preventing or suppressing, in a subject to expected to receive, receiving or having received an allofactor, the immune responses to said allofactor and/or the activation of CD4+ effector T-cells by said soluble allofactor and/or for inducing in said subject CD4+ regulatory T cells which are cytotoxic to cells presenting said soluble allofactor.

The present invention relates in one aspect to the use of at least one isolated immunogenic peptide comprising (i) a T-cell epitope derived from a soluble allofactor and (ii) a [CST]-(X)2-[CST] motif, more particularly a C-(X)2-[CST] or [CST]-(X)2-C motif, for the manufacture of a medicament for preventing or suppressing, in a subject expected to receive, receiving or having received said allofactor, the immune responses to said allofactor.

In a further aspect, the invention relates to the use of at least one isolated immunogenic peptide comprising (i) a T-cell epitope derived from a soluble allofactor and (ii) a [CST]-(X)2-[CST] motif, more particularly a C-(X)2-[CST] or [CST]-(X)2-C motif, for the manufacture of a medicament for preventing or suppressing, in a subject to receive, receiving or having received said allofactor, activation of CD4+ effector T-cells by said soluble allofactor.

In a further aspect, the invention also covers the use of at least one isolated immunogenic peptide comprising (i) a T-cell epitope derived from a soluble allofactor and (ii) a [CST]-(X)2-[CST] motif, more particularly a C-(X)2-[CST] or [CST]-(X)2-C motif, for the manufacture of a medicament for inducing, in a subject to receive, receiving or having received said allofactor, CD4+ regulatory T cells which are cytotoxic to cells presenting said soluble allofactor.

In any of the above uses said soluble allofactor may be a protein applied in replacement therapy, or a coagulation or fibrinolytic factor, or a hormone, or a cytokine or a growth factor, or an antibody used for therapeutic purposes.

In any of the above uses, the C-(X)2-[CST] or [CST]-(X)2-C motif in the immunogenic peptide may be adjacent to the T-cell epitope, or may be separated from the T-cell epitope by a linker. In particular embodiments, the linker consists of at most 7 amino acids.

In further embodiments of the immunogenic peptide for use in the applications described herein, the C-(X)2-[CST] or [CST]-(X)2-C motif does not naturally occur within a region of 11 amino acids N- or C-terminally adjacent to the T-cell epitope in the soluble allofactor from which the peptide is derived. In particular embodiments, the C-(X)2-[CST] or [CST]-(X)2-C motif is positioned N-terminally of the T-cell epitope. In further in particular embodiments, at least one X in said C-(X)2-[CST] or [CST]-(X)2-C motif is Gly, Ala, Ser or Thr; additionally or alternatively, at least one X is His or Pro. In particular embodiments at least one C in said C-(X)2-[CST] or [CST]-(X)2-C motif is methylated.

In particular embodiments of the immunogenic peptides envisaged for use in the above-described applications, the immunogenic peptide further comprises an endosomal targeting sequence. Any of the above immunogenic peptides may be produced by chemical synthesis or by recombinant expression.

A further aspect of the invention relates to methods for obtaining a population of soluble allofactor-specific regulatory T cells with cytotoxic properties, said methods comprising the steps of:
- providing peripheral blood cells;
- contacting said cells with an immunogenic peptide comprising (i) a T-cell epitope derived from a soluble allofactor and (ii) a C-(X)2-[CST] or [CST]-(X)2-C motif; and
- expanding said cells in the presence of IL-2.

A further embodiment of methods of the invention relates to obtaining a population of soluble allofactor-specific regulatory T cells with cytotoxic properties, such methods comprising the steps of:
- providing an immunogenic peptide comprising (i) a T-cell epitope derived from a soluble allofactor and (ii) a C-(X)2-[CST] or [CST]-(X)2-C motif;,
- administering said immunogenic peptide to a subject; and
- obtaining said population of soluble allofactor-specific regulatory T cells from said subject.

Populations of soluble allofactor-specific regulatory T cells with cytotoxic properties obtainable by the above methods are also part of the invention, as well as their use for the manufacture of a medicament for preventing or suppressing immune responses to soluble allofactors in a subject expected to receive, receiving or having received said allofactor.

A further aspect of the invention relates to isolated immunogenic peptides comprising a T-cell epitope from a soluble allofactor and, adjacent to said T-cell epitope or separated from said T-cell epitope by a linker, a C-(X)2-[CST] or [CST]-(X)2-C motif. More particularly, the invention provides immunogenic peptides of soluble allofactor epitopes, whereby the natural sequence of the soluble allofactor does not comprise the C-(X)2-[CST] or [CST]-(X)2-C within 11 amino acids N- or C-terminally adjacent to the epitope.

Yet another aspect of the invention relates to the use of at least one isolated immunogenic peptide comprising (i) a T-cell epitope derived from an anti-allofactor antibody idiotype and (ii) a [CST]-(X)2-[CST] motif, more particulary a C-(X)2-[CST] or [CST]-(X)2-C motif for the manufacture of a medicament for (substantially) eliminating allofactor-specific B cells in a subject having received said allofactor.

### FIGURE LEGENDS

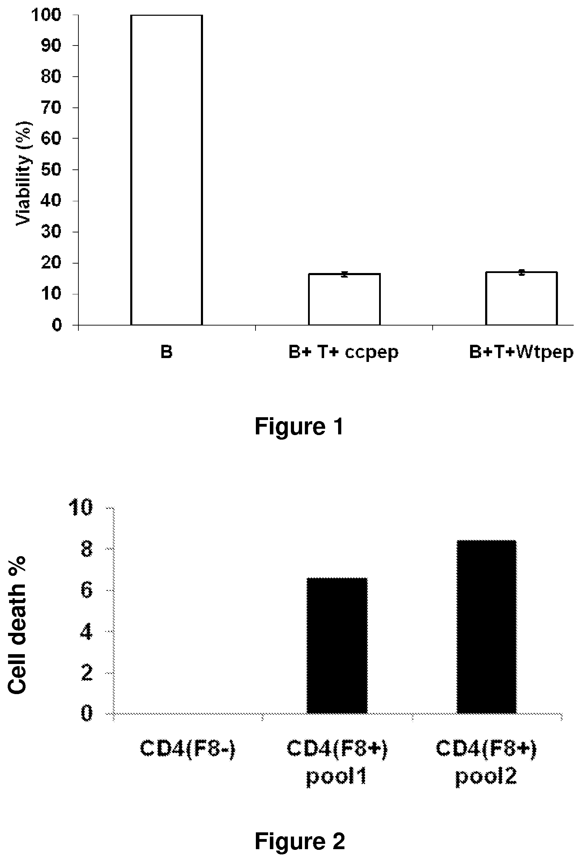

**Figure 1****.** The bars in this Figure illustrate the viability of murine splenic B cells incubated under different conditions and evaluated by Facs analysis of annexin V binding and 7-AAD (two markers of apoptosis).
   - B (wt-pep): murine splenic B cells incubated with natural (wild-type) T-cell epitope derived from human anti-fVIII antibody;
   - B (cc-pep): murine splenic B cells incubated with T-cell epitope derived from human anti-fVIII antibody, wherein said T-cell epitope is modified by attaching the amino acids CHGC to the N-terminus of the epitope;
   - B (cc-pep) + T (cc-pep): murine splenic B cells incubated with modified anti-fVIII antibody T-cell epitope and with T cells expanded with modified T-cell epitope (modification as in "B (cc-pep)");
   - B (wt-pep) + T (cc-pep): murine splenic B cells incubated with wild-type anti-fVIII antibody T-cell epitope and T cells expanded with the corresponding modified T-cell epitope.
      See Example 1 for details on the T-cell epitopes.
**Figure 2****.** Apoptosis of allofactor-specific effector T-cells by cytolytic CD4+ T-cells induced by a T-cell epitope derived from said allofactor and modified by attaching a thioreductase motif. The allofactor used was factor VIII. See Example 5 for more details.

### DETAILED DESCRIPTION OF THE INVENTION

### Definitions

The term **"peptide"** when used herein refers to a molecule comprising an amino acid sequence of between 2 and 200 amino acids, connected by peptide bonds, but which can in a particular embodiment comprise non-amino acid structures (like for example a linking organic compound). Peptides according to the invention can contain any of the conventional 20 amino acids or modified versions thereof, or can contain non-naturally occurring amino acids incorporated by chemical peptide synthesis or by chemical or enzymatic modification.

The term **"epitope"** when used herein refers to one or several portions (which may define a conformational epitope) of a protein or factor which is/are specifically recognised and bound by an antibody or a portion thereof (Fab', Fab2', etc.) or a receptor presented at the cell surface of a B or T cell lymphocyte, and which is able, by said binding, to induce an immune response.

The term **"antigen"** when used herein refers to a structure of a macromolecule comprising one or more hapten(s) (eliciting an immune response only when attached to a carrier) and/or comprising one or more T cell epitopes. Typically, said macromolecule is a protein or peptide (with or without polysaccharides) or made of proteic composition and comprises one or more epitopes; said macromolecule can herein alternatively be referred to as **"antigenic protein"** or **"antigenic peptide".**

The term **"allofactor"** refers to a protein, peptide or factor (i.e., any molecule) displaying polymorphism when compared between 2 individuals of the same species, and, more in general, any protein, peptide or factor that induces an (alloreactive) immune response in the subject receiving the allofactor.

The term **"alloreactivity"** refers to an immune response in a subject receiving an allofactor, with said immune response in principle being directed towards allelic differences between the administered allofactor and the recipient's own version of the factor. Alloreactivity applies to antibodies and to T cells.

The term **"T cell epitope"** or **"T-cell epitope"** in the context of the present invention refers to a dominant, sub-dominant or minor T cell epitope, i.e., a part of an antigenic protein or factor that is specifically recognized and bound by a receptor at the cell surface of a T lymphocyte. Whether an epitope is dominant, sub-dominant or minor depends on the immune reaction elicited against the epitope. Dominance depends on the frequency at which such epitopes are recognised by T cells and able to activate them, among all the possible T cell epitopes of a protein. In particular, a T cell epitope is an epitope bound by MHC class I or MHC class II molecules.

The term **"MHC"** refers to "major histocompatibility antigen". In humans, the MHC genes are known as HLA ("human leukocyte antigen") genes. Although there is no consistently followed convention, some literature uses HLA to refer to HLA protein molecules, and MHC to refer to the genes encoding the HLA proteins. As such the terms "MHC" and "HLA" are equivalents when used herein. The HLA system in man has its equivalent in the mouse, i.e., the H2 system. The most intensely-studied HLA genes are the nine so-called classical MHC genes: HLA-A, HLA-B, HLA-C, HLA-DPA1, HLA-DPB1, HLA-DQA1, HLA-DQB1, HLA-DRA, and HLA-DRB1. In humans, the MHC is divided into three regions: Class I, II, and III. The A, B, and C genes belong to MHC class I, whereas the six D genes belong to class II. MHC class I molecules are made of a single polymorphic chain containing 3 domains (alpha 1, 2 and 3), which associates with beta 2 microglobulin at cell surface. Class II molecules are made of 2 polymorphic chains, each containing 2 chains (alpha 1 and 2, and beta 1 and 2).

Class I MHC molecules are expressed on virtually all nucleated cells. Peptide fragments presented in the context of class I MHC molecules are recognised by CD8+ T lymphocytes (cytotoxic T lymphocytes or CTLs). CD8+ T lymphocytes frequently mature into cytotoxic effectors which can lyse cells bearing the stimulating antigen. Class II MHC molecules are expressed primarily on activated lymphocytes and antigen-presenting cells. CD4+ T lymphocytes (helper T lymphocytes or HTLs) are activated with recognition of a unique peptide fragment presented by a class II MHC molecule, usually found on an antigen presenting cell like a macrophage or dendritic cell. CD4+ T lymphocytes proliferate and secrete cytokines that either support an antibody-mediated response through the production of IL-4 and IL-10 or support a cell-mediated response through the production of IL-2 and IFN-gamma.

Functional HLAs are characterised by a deep binding groove to which endogenous as well as foreign, potentially antigenic peptides bind. The groove is further characterised by a well-defined shape and physico-chemical properties. HLA class I binding sites are closed, in that the peptide termini are pinned down into the ends of the groove. They are also involved in a network of hydrogen bonds with conserved HLA residues. In view of these restraints, the length of bound peptides is limited to 8-10 residues. However, it has been demonstrated that peptides of up to 12 amino acid residues are also capable of binding HLA class I. Superposition of the structures of different HLA complexes confirmed a general mode of binding wherein peptides adopt a relatively linear, extended conformation.

In contrast to HLA class I binding sites, class II sites are open at both ends. This allows peptides to extend from the actual region of binding, thereby "hanging out" at both ends. Class II HLAs can therefore bind peptide ligands of variable length, ranging from 9 to more than 25 amino acid residues. Similar to HLA class I, the affinity of a class II ligand is determined by a "constant" and a "variable" component. The constant part again results from a network of hydrogen bonds formed between conserved residues in the HLA class II groove and the main-chain of a bound peptide. However, this hydrogen bond pattern is not confined to the N-and C-terminal residues of the peptide but distributed over the whole chain. The latter is important because it restricts the conformation of complexed peptides to a strictly linear mode of binding. This is common for all class II allotypes. The second component determining the binding affinity of a peptide is variable due to certain positions of polymorphism within class II binding sites. Different allotypes form different complementary pockets within the groove, thereby accounting for subtype-dependent selection of peptides, or specificity. Importantly, the constraints on the amino acid residues held within class II pockets are in general "softer" than for class I. There is much more cross reactivity of peptides among different HLA class II allotypes. The sequence of the +/- 9 amino acids of an MHC class II T cell epitope that fit in the groove of the MHC II molecule are usually numbered P1 to P9. Additional amino acids N-terminal of the epitope are numbered P-1, P-2 and so on, amino acids C-terminal of the epitope are numbered P+1, P+2 and so on.

The term **"organic compound having a reducing activity"** when used herein refers to compounds, more in particular amino acid sequences, capable of reducing disulfide bonds in proteins. An alternatively used term for such an amino acid sequence is "redox motif".

The term **"therapeutically effective amount"** refers to an amount of the peptide of the invention or derivative thereof, which produces the desired therapeutic or preventive effect in a patient. For example, in reference to a disease or disorder, it is the amount which reduces to some extent one or more symptoms of the disease or disorder, and more particularly returns to normal, either partially or completely, the physiological or biochemical parameters associated with or causative of the disease or disorder. According to one particular embodiment of the present invention, the therapeutically effective amount is the amount of the peptide of the invention or derivative thereof, which will lead to an improvement or restoration of the normal physiological situation. For instance, when used to therapeutically treat a mammal affected by an immune disorder, it is a daily amount peptide/kg body weight of the said mammal. Alternatively, where the administration is through gene-therapy, the amount of naked DNA or viral vectors is adjusted to ensure the local production of the relevant dosage of the peptide of the invention, derivative or homologue thereof.

The term **"natural"** when used herein referring to a sequence n relates to the fact that the (amino acid or nucleotide) sequence is identical to a naturally occurring sequence or is identical to part of such naturally occurring sequence. In contrast therewith the term **"artificial"** refers to a sequence which as such does not occur in nature. Unless otherwise specified, the terms natural and artificial referring to a sequence thus exclusively relate to a particular amino acid (or nucleotide) sequence (e.g. the sequence of the immunogenic peptide, a sequence comprised within the immunogenic peptide, an epitope sequence) and do not refer to the nature of the immunogenic peptide as such.

Optionally, an artificial sequence is obtained from a natural sequence by limited modifications such as changing one or more amino acids within the naturally occurring sequence or by adding amino acids N- or C-terminally of a naturally occurring sequence. Amino acids are referred to herein with their full name, their three-letter abbreviation or their one letter abbreviation.

Motifs of amino acid sequences are written herein according to the format of Prosite (Hulo et al. (2006) Nucleic Acids Res. 34 (Database issue D227-D230). The symbol X is used for a position where any amino acid is accepted. Alternatives are indicated by listing the acceptable amino acids for a given position, between square brackets ('[ ]'). For example: [CST] stands for an amino acid selected from Cys, Ser or Thr. Amino acids which are excluded as alternatives are indicated by listing them between curly brackets ('{ }'). For example: {AM} stands for any amino acid except Ala and Met. The different elements in a motif are separated from each other by a hyphen - . Repetition of an identical element within a motif can be indicated by placing behind that element a numerical value or a numerical range between parentheses. For example: X(2) corresponds to X-X, X(2, 4) corresponds to X-X or X-X-X or X-X-X-X , A(3) corresponds to A-A-A.

The term **"homologue"** when used herein with reference to the epitopes used in the context of the invention, refer to molecules having at least 50%, at least 70%, at least 80%, at least 90%, at least 95% or at least 98% amino acid sequence identity with the naturally occurring epitope, thereby maintaining the ability of the epitope to bind an antibody or cell surface receptor of a B and/or T cell. Particular embodiments of homologues of an epitope correspond to the natural epitope modified in at most three, more particularly in at most two, most particularly in one amino acid.

The term **"derivative"** when used herein with reference to the peptides of the invention refers to molecules which contain at least the peptide active portion (i.e. capable of eliciting cytolytic CD4+ T cell activity) and, in addition thereto comprises a complementary portion which can have different purposes such as stabilising the peptides or altering the pharmacokinetic or pharmacodynamic properties of the peptide.

The term **"sequence identity"** of two sequences when used herein relates to the number of positions with identical nucleotides or amino acids divided by the number of nucleotides or amino acids in the shorter of the sequences, when the two sequences are aligned. In particular embodiments, said sequence identity is from 70% to 80%, from 81% to 85%, from 86% to 90%, from 91 % to 95%, from 96% to 100%, or 100%.

The terms **"peptide-encoding polynucleotide (or nucleic acid)"** and **"polynucleotide (or nucleic acid) encoding peptide"** when used herein refer to a nucleotide sequence, which, when expressed in an appropriate environment, results in the generation of the relevant peptide sequence or a derivative or homologue thereof. Such polynucleotides or nucleic acids include the normal sequences encoding the peptide, as well as derivatives and fragments of these nucleic acids capable of expressing a peptide with the required activity. According to one embodiment, the nucleic acid encoding the peptides according to the invention or fragment thereof is a sequence encoding the peptide or fragment thereof originating from a mammal or corresponding to a mammalian, most particularly a human peptide fragment.

The present invention finds its origin in the observation that CD4+ T-cells isolated from naïve mice immunised with a human antibody, and subsequently stimulated with modified T-cell epitope derived from said antibody, were able to drive naïve B-cells presenting said T-cell epitope (natural or modified) into apoptosis. The modification of the T-cell epitope existed therein that it was extended by a motif capable of catalysing disulfide-bridge shuffling in proteins, i.e., a sequence containing thioreductase activity (hereinafter also simply referred to as redox motif).

Thus, the present invention provides ways to prevent and/or suppress immune responses to proteins derived from soluble allofactors as used in, e.g., replacement therapy. In particular, the invention provides ways to prevent the development of and/or suppress a CD4+ effector T cells (alternatively referred to as bystander T cells) response. Instead CD4+ regulatory T cells are induced which are capable of specifically inducing apoptosis of APCs (such a B cells) presenting T cell epitopes processed from soluble allofactors, thereby preventing the formation of specific antibodies.

The compounds used to achieve the above are immunogenic peptides encompassing the sequence of a T cell epitope derived from soluble allofactors (e.g. by processing) and presented in the context of MHC class II determinants attached to a redox motif such as C-(X)2-C. The T cell epitope modified in this way alters the activation pattern and function of CD4+ T cells, either *de novo* from naïve T cells in a prevention setting, or by modifying the properties of memory T cells, both resulting in a potent capacity to induce apoptosis of APC. Thereby the antibody and cellular responses towards soluble allofactors are prevented and/or suppressed. More specifically, the elimination of an APC (dendritic cells or macrophages, or B cells, in the setting of primary and secondary immune responses, respectively) presenting MHC class II bound peptides processed from soluble allofactors results in tolerance induction to said soluble allofactors. Hence, an important side-effect of e.g. replacement therapy is eliminated by using the above-described compounds.

Thus, in a one aspect the invention relates to the use of at least one isolated immunogenic peptide according to the invention for preventing or suppressing, in a subject expected to receive, receiving or having received a soluble allofactor, the immune responses to said soluble allofactor. More particularly, the invention relates to the use of at least one isolated immunogenic peptide comprising (i) a T-cell epitope derived from a soluble allofactor and (ii) a C-(X)2-[CST] or [CST]-(X)2-C motif, for the manufacture of a medicament for preventing or suppressing, in a subject expected to receive, receiving or having received said soluble allofactor, the immune responses to said soluble allofactor. Hence, said immunogenic peptide or the medicament comprising it can be used for prior or prophylactic treatment or immunisation of a subject that will receive (and accordingly is expected to receive)(or is receiving) said soluble (therapeutic) allofactor in order to suppress, avoid, reduce partially or totally, or eliminate (partially or totally) immune response(s) induced by the subsequently administered soluble allofactor. Likewise, said immunogenic peptide or the medicament comprising it can be used for therapeutic treatment or immunisation of a subject that has received (or is receiving) said soluble (therapeutic) allofactor in order to suppress, reduce partially or totally, or eliminate (partially or totally) ongoing immune response(s) induced by the administration of said soluble allofactor.

In a further aspect, the invention relates to the use of at least one isolated immunogenic peptide comprising (i) a T-cell epitope derived from a soluble allofactor and (ii) a C-(X)2-[CST] or [CST]-(X)2-C motif, for the manufacture of a medicament for preventing, in a subject to receive, receiving or having received said soluble allofactor, activation of CD4+ effector T-cells by said soluble allofactor.

In a further aspect, the invention also covers the use of at least one isolated immunogenic peptide comprising (i) a T-cell epitope derived from a soluble allofactor and (ii) a C-(X)2-[CST] or [CST]-(X)2-C motif, for the manufacture of a medicament for inducing, in a subject to receive, receiving or having received said soluble allofactor, CD4+ regulatory T cells which are cytotoxic to cells presenting said soluble allofactor.

In the above aspects of the invention, the immunogenic peptide or the medicament comprising it can be used for prior or prophylactic treatment or immunisation of in a subject which will receive (or is receiving) said soluble (therapeutic) allofactor in order to suppress, avoid, reduce partially or totally, or eliminate (partially or totally) a normally expected activation in the recipient of CD4+ effector T-cells towards the soluble allofactor following or subsequent to the actual administration of said soluble allofactor. Likewise, the one or more immunogenic peptides or the medicaments comprising them can be used for therapeutic treatment or immunisation of a subject which has received (or is receiving) said soluble (therapeutic) allofactor in order to suppress, reduce partially or totally, or eliminate (partially or totally) activation in the recipient of CD4+ effector T-cells and/or CD8+ T-cells towards the soluble allofactor concurrent with or after the actual administration of said soluble allofactor. Alternatively, or concurrently with any of the above, the immunogenic peptide or the medicament comprising it can be used for prior or prophylactic treatment or immunisation of a in a subject which will receive (or is receiving) said soluble (therapeutic) allofactor in order to induce a normally unexpected activation in the recipient of soluble allofactor-specific CD4+ regulatory T-cells capable of killing cells presenting soluble allofactor antigen(s) following or subsequent to the actual administration of said soluble allofactor. Likewise, said immunogenic peptide or the medicament comprising it can be used for therapeutic treatment or immunisation of a subject which has received (or is receiving) said soluble (therapeutic) allofactor in order to induce activation in said subject of soluble allofactor-specific CD4+ regulatory T-cells capable of killing cells presenting said soluble allofactor. Said induction may happen concurrent with or after the actual administration of said soluble allofactor.

In any of the uses described hereinabove, the subject or recipient is a mammal, in particular a (non-human) primate or a human.

In any of the above uses the soluble allofactor may be a protein applied in replacement therapy, or a coagulation or fibrinolytic factor, or a hormone, or a cytokine or a growth factor, or an antibody used for therapeutic purposes. A non-limiting list of possible allofactors includes factor VIII, factor IX, staphylokinase, growth hormone, insulin, cytokines and growth factors (such as interferon-alpha, interferon-gamma, GM-CSF and G-CSF), antibodies for the modulation of immune responses (including anti-IgE antibodies in allergic diseases, anti-CD3 and anti-CD4 antibodies in graft rejection and a variety of autoimmune diseases, anti-CD20 antibodies in non-Hodgkin lymphomas), and erythropoietin in renal insufficiency.

Cytotoxic regulatory T cells elicited by the immunogenic peptides of the present invention can suppress immune responses to even complex soluble (therapeutic) allofactors. A minimum requirement for such cells to be activated is to recognise a cognate peptide presented by MHC class II determinants, leading to apoptosis of the APC, thereby suppressing the responses of T cells (both CD4+ and CD8+ T cells) to all T cell epitopes presented by the APC. An additional mechanism by which cytotoxic regulator T cells can suppress the overall immune response towards complex antigens is by suppressing the activation of bystander T cells.

It is envisaged that there are situations in which more than one soluble allofactor antigen contributes to an immune response to a soluble allofactor. Under such circumstances, the same APC may not present all relevant soluble allofactor antigens, as some of such antigens may be taken up by potentially different APCs. It is therefore anticipated that combination of two or more immunogenic peptides may be used for the prevention and suppression of immune responses to said soluble allofactor.

A further aspect of the invention relates to methods such as those described hereinabove, wherein said immunogenic peptide is replaced by CD4+ regulatory T-cells primed with said immunogenic peptide. In yet a further aspect methods such as those described above are envisaged wherein the immunogenic peptide is replaced by a nucleotide sequence encoding the immunogenic peptide (e.g. in the form of naked DNA or a viral vector to be administered to an individual instead of the immunogenic peptide). In addition, a combination of multiple immunogenic peptides, i.e. more than 1 (e.g., 2, 3, 4, 5, 6, 7, 8, 9, 10 or more), can be used in any of the above. These aspects of the invention, as well as the further modification of the immunogenic peptide are described in detail hereafter.

The present invention is based upon the finding that an immunogenic peptide, comprising a T cell epitope derived from a soluble (therapeutic) allofactor and a peptide sequence, having reducing activity is capable of generating a population of CD4+ regulatory T cells, which have a cytotoxic effect on antigen presenting cells. It is additionally based upon the finding that such immunogenic peptide is capable of preventing activation of soluble allofactor-specific CD8+ T cells and/or CD4+ effector T cells.

Accordingly, the invention relates to immunogenic peptides, which comprise at least one T-cell epitope of a soluble (therapeutic) allofactor with a potential to trigger an immune reaction, coupled to an organic compound having a reducing activity, such as a thioreductase sequence motif. The T cell epitope and the organic compound are optionally separated by a linker sequence. In further optional embodiments the immunogenic peptide additionally comprises an endosome targeting sequence (e.g. late endosomal targeting sequence) and/or additional "flanking" sequences.

The immunogenic peptides of the invention can be schematically represented as A-L- B or B-L-A, wherein A represents a T-cell epitope of an antigen (self or non-self) with a potential to trigger an immune reaction, L represents a linker and B represents an organic compound having a reducing activity.

The reducing activity of an organic compound can be assayed for its ability to reduce a sulfhydryl group such as in the insulin solubility assay known in the art, wherein the solubility of insulin is altered upon reduction, or with a fluorescence-labelled insulin. The reducing organic compound may be coupled at the amino-terminus side of the T-cell epitope or at the carboxy-terminus of the T-cell epitope.

Generally the organic compound with reducing activity is a peptide sequence. Peptide fragments with reducing activity are encountered in thioreductases which are small disulfide reducing enzymes including glutaredoxins, nucleoredoxins, thioredoxins and other thiol/disulfide oxidoreductases They exert reducing activity for disulfide bonds on proteins (such as enzymes) through redox active cysteines within conserved active domain consensus sequences: C-X(2)-C, C-X(2)-S, C-X(2)-T, S-X(2)-C, T-X(2)-C (Fomenko et al. (2003) Biochemistry 42, 11214-11225), in which X stands for any amino acid. Such domains are also found in larger proteins such as protein disulfide isomerase (PDI) and phosphoinositide-specific phospholipase C.

Accordingly, in particular embodiments, immunogenic peptides according to the present invention comprise as redox motif the thioreductase sequence motif [CST]-X(2)-[CST], in a further embodiment thereto, said [CST]-X(2)-[CST] motif is positioned N-terminally of the T-cell eptiope. More specifically, in said redox motif at least one of the [CST] positions is occupied by a Cys; thus the motif is either C-X(2)-[CST] or [CST]-X(2)-C. In the present application such a tetrapeptide will be referred to as "the motif". In particular embodiments peptides of the invention contain the sequence motif C-X(2)-[CS] or [CS]-X(2)-C. In more particular embodiments peptides contain the sequence motif C-X(2)-S, S-X(2)-C or C-X(2)-C.

As explained in detail further on, the immunogenic peptides of the present invention can be made by chemical synthesis, which allows the incorporation of non-natural amino acids. Accordingly, in the motif of reducing compounds according to particular embodiments of the present invention, C represents either cysteine or another amino acids with a thiol group such as mercaptovaline, homocysteine or other natural or non-natural amino acids with a thiol function. In order to have reducing activity, the cysteines present in the motif should not occur as part of a cystine disulfide bridge. Nevertheless, the motif may comprise modified cysteines such as methylated cysteine, which is converted into cysteine with free thiol groups *in vivo.*

Each of the amino acids X in the C-X(2)-[CST] or [CST]-X(2)-C motif of particular embodiments of the immunogenic peptides of the invention can be any natural amino acid, including S, C, or T or can be a non-natural amino acid. In particular embodiments X is an amino acid with a small side chain such as Gly, Ala, Ser or Thr. In further particular embodiments, X is not an amino acid with a bulky side chain such as Tyr. In further particular embodiments at least one X in the [CST]-X(2)-[CST] motif is His or Pro.

In the immunogenic peptides for use in the methods of the present invention comprising the (redox) motif described above, the motif is located such that, when the epitope fits into the MHC groove, the motif remains outside of the MHC binding groove. The motif is placed either immediately adjacent to the epitope sequence within the peptide, or is separated from the T cell epitope by a linker. More particularly, the linker comprises an amino acid sequence of 7 amino acids or less. Most particularly, the linker comprises 1, 2, 3, or 4 amino acids. Alternatively, a linker may comprise 6, 8 or 10 amino acids. Typical amino acids used in linkers are serine and threonine. Example of peptides with linkers in accordance with the present invention are CXXC-G-epitope (SEQ ID NO:6), CXXC-GG-epitope (SEQ ID NO:7), CXXC-SSS-epitope (SEQ ID NO:8), CXXC-SGSG-epitope (SEQ ID NO:9) and the like.

In those particular embodiments of the peptides of the invention where the motif sequence is adjacent to the epitope sequence this is indicated as position P-4 to P-1 or P+1 to P+4 compared to the epitope sequence. Apart from a peptide linker other organic compounds can be used as linker to link the parts of the immunogenic peptide to each other.

The immunogenic peptides for use in the methods and applications of the present invention can further comprise additional short amino acid sequences N or C-terminally of the (artificial) sequence comprising the T cell epitope and the reducing compound (motif). Such an amino acid sequence is generally referred to herein as a 'flanking sequence'. A flanking sequence can be positioned N- and/or C-terminally of the redox motif and/or of the T-cell epitope in the immunogenic peptide. When the immunogenic peptide comprises an endosomal targeting sequence, a flanking sequence can be present between the epitope and an endosomal targeting sequence and/or between the reducing compound (e.g. motif) and an endosomal targeting sequence. More particularly a flanking sequence is a sequence of up to 10 amino acids, or of in between 1 and 7 amino acids, such as a sequence of 2 amino acids.

In particular embodiments of the invention, the redox motif in the immunogenic peptide is located N-terminally from the epitope.

In further particular embodiments, where the redox motif present in the immunogenic peptide contains one cysteine, this cysteine is present in the motif in the position most remote from the epitope, thus the motif occurs as C-X(2)-[ST] or C-X(2)-S N-terminally of the epitope or occurs as [ST]-X(2)-C or S-X(2)-C carboxy-terminally of the epitope.

In certain embodiments of the present invention, immunogenic peptides are provided comprising one epitope sequence and a motif sequence. In further particular embodiments, the motif occurs several times (1, 2, 3, 4 or even more times) in the peptide, for example as repeats of the motif which can be spaced from each other by one or more amino acids (e.g. CXXC X CXXC X CXXC; SEQ ID NO:10), as repeats which are adjacent to each other (CXXC CXXC CXXC; SEQ ID NO:11) or as repeats which overlap with each other CXXCXXCXXC (SEQ ID NO:12) or CXCCXCCXCC (SEQ ID NO:13)). Alternatively, one or more motifs are provided at both the N and the C terminus of the T cell epitope sequence. Other variations envisaged for the immunogenic peptides of the present invention include peptides containing repeats of a T cell epitope sequence or multiple different T-cell epitopes wherein each epitope is preceded and/or followed by the motif (e.g. repeats of "motif-epitope" or repeats of "motif-epitope-motif"). Herein the motifs can all have the same sequence but this is not obligatory. It is noted that repetitive sequences of peptides which comprise an epitope which in itself comprises the motif will also result in a sequence comprising both the 'epitope' and a 'motif'. In such peptides, the motif within one epitope sequence functions as a motif outside a second epitope sequence. In particular embodiments however, the immunogenic peptides of the present invention comprise only one T cell epitope.

As described above the immunogenic peptides for use in methods according to the invention comprise, in addition to a reducing compound/motif, a T cell epitope derived from a soluble allofactor. A T cell epitope in a protein sequence can be identified by functional assays and/or one or more *in silico* prediction assays. The amino acids in a T cell epitope sequence are numbered according to their position in the binding groove of the MHC proteins. In particular embodiments, the T-cell epitope present within the peptides of the invention consists of between 8 and 25 amino acids, yet more particularly of between 8 and 16 amino acids, yet most particularly consists of 8, 9, 10, 11, 12, 13, 14, 15 or 16 amino acids. In a more particular embodiment, the T cell epitope consists of a sequence of 9 amino acids. In a further particular embodiment, the T-cell epitope is an epitope, which is presented to T cells by MHC-class II molecules. In particular embodiments of the present invention, the T cell epitope sequence is an epitope sequence which fits into the cleft of an MHC II protein, more particularly a nonapeptide fitting into the MHC II cleft. The T cell epitope of the immunogenic peptides of the invention can correspond either to a natural epitope sequence of a protein or can be a modified version thereof, provided the modified T cell epitope retains its ability to bind within the MHC cleft, similar to the natural T cell epitope sequence. The modified T cell epitope can have the same binding affinity for the MHC protein as the natural epitope, but can also have a lowered affinity. In particular embodiments the binding affinity of the modified peptide is no less than 10-fold less than the original peptide, more particularly no less than 5 times less. It is a finding of the present invention that the peptides of the present invention have a stabilizing effect on protein complexes. Accordingly, the stabilizing effect of the peptide-MHC complex compensates for the lowered affinity of the modified epitope for the MHC molecule.

In particular embodiments, the immunogenic peptides for use in the methods of the invention further comprise an amino acid sequence (or another organic compound) facilitating uptake of the peptide into (late) endosomes for processing and presentation within MHC class II determinants. The late endosome targeting is mediated by signals present in the cytoplasmic tail of proteins and correspond to well-identified peptide motifs such as the dileucine-based [DE]XXXL[LI] (SEQ ID NO:14) or DXXLL (SEQ ID NO:15) motif (e.g. DXXXLL; SEQ ID NO:16), the tyrosine-based YXXØ motif or the so-called acidic cluster motif. The symbol Ø represents amino acid residues with a bulky hydrophobic side chains such as Phe, Tyr and Trp. The late endosome targeting sequences allow for processing and efficient presentation of the antigen-derived T cell epitope by MHC-class II molecules. Such endosomal targeting sequences are contained, for example, within the gp75 protein (Vijayasaradhi et al. (1995) J Cell Biol 130, 807-820), the human CD3 gamma protein, the HLA-BM ß (Copier et al. (1996) J. Immunol. 157, 1017-1027), the cytoplasmic tail of the DEC205 receptor (Mahnke et al. (2000) J Cell Biol 151, 673-683). Other examples of peptides which function as sorting signals to the endosome are disclosed in the review of Bonifacio and Traub (2003) Annu. Rev. Biochem. 72, 395-447. Alternatively, the sequence can be that of a subdominant or minor T cell epitope from a protein, which facilitates uptake in late endosome without overcoming the T cell response towards the soluble allofactor-derived T cell epitope.

The immunogenic peptides for use in the methods of the invention can be generated by coupling a reducing compound, more particularly a reducing motif as described herein, N-terminally or C-terminally to a T-cell epitope of the soluble (therapeutic) allofactor (either directly adjacent thereto or separated by a linker). Moreover the T cell epitope sequence of the immunogenic peptide and/or the redox motif can be modified and/or one or more flanking sequences and/or a targeting sequence can be introduced (or modified), compared to the naturally occurring T-cell epitope sequence. Accordingly, the resulting sequence of the immunogenic peptide will in most cases differ from the sequence of the soluble allofactor protein of interest. In these cases, the immunogenic peptides of the invention are peptides with an 'artificial', non-naturally occurring sequence.

The immunogenic peptides for use in the context of the invention can vary substantially in length, e.g. from about 12-13 amino acids (a T-cell epitope of 8-9 amino acids and the 4-amino acid redox motif) to up to 50 or more amino acids. For example, an immunogenic peptide according to the invention may comprise an endosomal targeting sequence of 40 amino acids, a flanking sequence of about 2 amino acids, a motif as described herein of 4 amino acids, a linker of 4 amino acids and a T cell epitope peptide of 9 amino acids. In particular embodiments, the immunogenic peptides of the invention consist of between 12 amino acids and 20 up to 25, 30, 50, 75, 100 or 200 amino acids. In a more particular embodiment, the peptides consist of between 10 and 20 amino acids. More particularly, where the reducing compound is a redox motif as described herein, the length of the immunogenic peptide comprising the epitope and motif optionally connected by a linker is 19 amino acids or less, e.g., 12, 13, 14, 15, 16, 17, 18 or 19 amino acids.

As detailed above, the immunogenic peptides for use in the context of the present invention comprise a reducing motif as described herein linked to a T cell epitope sequence. According to a particular embodiment the T-cell epitopes are derived from soluble allofactors which do not comprise within their native natural sequence an amino acid sequence with redox properties within a sequence of 11 amino acids N- or C- terminally adjacent to the T-cell epitope of interest. Most particularly, the invention encompasses generating immunogenic peptides from soluble allofactors which do not comprise a sequence selected from C-X(2)-S, S-X(2)-C, C-X(2)-C, S-X(2)-S, C-X(2)-T, T-X(2)-C within a sequence of 11 amino acids N- or C-terminally adjacent to the epitope sequence. In further particular embodiments, the present invention provides immunogenic peptides of soluble allofactors which do not comprise the above-described amino acid sequences with redox properties within their sequence.

In further particular embodiments, the immunogenic peptides of the invention are peptides comprising T cell epitopes whereby the epitopes do not comprise an amino acid sequence with redox properties within their natural sequence. However, in alternative embodiments, a T cell epitope binding to the MHC cleft may comprise a redox motif such as described herein within its epitope sequence; the immunogenic peptides according to the invention comprising such T-cell epitope must further comprise another redox motif coupled (adjacent of separated by a linker) N- or C-terminally to the epitope such that the attached motif can ensure the reducing activity (contrary to the motif present in the epitope, which is buried within the cleft).

Another aspect of the present invention relates to methods for generating immunogenic peptides of the present invention described herein. Such methods include the identification of T-cell epitopes in a soluble allofactor of interest; ways for *in vitro* and *in silico* identification T-cell epitopes are known in the art and some aspects are elaborated upon hereafter. The generated immunogenic peptides are optionally assessed for the capability to induce soluble allofactor-specific CD4+ regulatory T cells which are cytotoxic for cells presenting (parts of) the soluble allofactor of interest.

Immunogenic peptides according to the invention are generated starting from T cell epitope(s) of the soluble allofactor(s) of interest. In particular, the T-cell epitope used may be a dominant T-cell epitope. The identification and selection of a T-cell epitope from a soluble allofactor, for use in the context of the present invention is performed by methods known to a person skilled in the art. For instance, peptide sequences isolated from a soluble allofactor are tested by, for example, T cell biology techniques, to determine whether the peptide sequences elicit a T cell response. Those peptide sequences found to elicit a T cell response are defined as having T cell stimulating activity. Human T cell stimulating activity can further be tested by culturing T cells obtained from an individual sensitised to a soluble allofactor with a peptide/epitope derived from the soluble allofactor and determining whether proliferation of T cells occurs in response to the peptide/epitope as measured, e.g., by cellular uptake of tritiated thymidine. Stimulation indices for responses by T cells to peptides/epitopes can be calculated as the maximum CPM in response to a peptide/epitope divided by the control CPM. A T cell stimulation index (S.I.) equal to or greater than two times the background level is considered "positive." Positive results are used to calculate the mean stimulation index for each peptide/epitope for the group of peptides/epitopes tested. Non-natural (or modified) T-cell epitopes can further optionally be tested for their binding affinity to MHC class II molecules. The binding of non-natural (or modified) T-cell epitopes to MHC class II molecules can be performed in different ways. For instance, soluble HLA class II molecules are obtained by lysis of cells homozygous for a given class II molecule. The latter is purified by affinity chromatography. Soluble class II molecules are incubated with a biotin-labelled reference peptide produced according to its strong binding affinity for that class II molecule. Peptides to be assessed for class II binding are then incubated at different concentrations and their capacity to displace the reference peptide from its class II binding is calculated by addition of neutravidin. Methods can be found in for instance Texier et al. (2000) J. Immunology 164, 3177-3184). The immunogenic peptides of the invention have a mean T cell stimulation index of greater than or equal to 2.0. An immunogenic peptide having a T cell stimulation index of greater than or equal to 2.0 is considered useful as a prophylactic or therapeutic agent. More particularly, immunogenic peptides according to the invention have a mean T cell stimulation index of at least 2.5, at least 3.5, at least 4.0, or even at least 5.0. In addition, such peptides typically have a positivity index (P.I.) of at least about 100, at least 150, at least about 200 or at least about 250. The positivity index for a peptide is determined by multiplying the mean T cell stimulation index by the percent of individuals, in a population of individuals sensitive to a soluble allofactor (e. g., at least 9 individuals, at least 16 individuals or at least 29 or 30, or even more), who have T cells that respond to the peptide (thus corresponding to the SI multiplied by the promiscuous nature of the peptide/epitope). Thus, the positivity index represents both the strength of a T cell response to a peptide (S.I.) and the frequency of a T cell response to a peptide in a population of individuals sensitive to a soluble allofactor. In order to determine optimal T cell epitopes by, for example, fine mapping techniques, a peptide having T cell stimulating activity and thus comprising at least one T cell epitope as determined by T cell biology techniques is modified by addition or deletion of amino acid residues at either the N- or C-terminus of the peptide and tested to determine a change in T cell reactivity to the modified peptide. If two or more peptides which share an area of overlap in the native protein sequence are found to have human T cell stimulating activity, as determined by T cell biology techniques, additional peptides can be produced comprising all or a portion of such peptides and these additional peptides can be tested by a similar procedure. Following this technique, peptides are selected and produced recombinantly or synthetically. T cell epitopes or peptides are selected based on various factors, including the strength of the T cell response to the peptide/epitope (e.g., stimulation index) and the frequency of the T cell response to the peptide in a population of individuals.

Candidate antigens can be screened by one or more *in vitro* algorithms to identify a T cell epitope sequence within an antigenic protein. Suitable algorithms are described for example in Zhang et al. (2005) Nucleic Acids Res 33, W180-W183 ( PREDBALB); Salomon & Flower (2006) BMC Bioinformatics 7, 501 (MHCBN); Schuler et al. (2007) Methods Mol Biol. 409, 75-93 (SYFPEITHI); Dönnes & Kohlbacher (2006) Nucleic Acids Res. 34, W194-W197 (SVMHC); Kolaskar & Tongaonkar (1990) FEBS Lett. 276, 172-174 and Guan et al. (2003) Appl Bioinformatics 2, 63-66 (MHCPred). More particularly, such algorithms allow the prediction within an antigenic protein of one or more nonapeptide sequences which will fit into the groove of an MHC II molecule.

The immunogenic peptides for use in the context of the present invention can be produced by recombinant expression in, e.g., bacterial cells (e.g. *Escherichia coli)*, yeast cells (e.g., *Pichia* species, *Hansenula* species, *Saccharomyces* or *Schizosaccharomyces* species), insect cells (e.g. from *Spodoptera frugiperda* or *Trichoplusia ni*), plant cells or mammalian cells (e.g., CHO, COS cells). The construction of the therefore required suitable expression vectors (including further information such as promoter and termination sequences) involves meanwhile standard recombinant DNA techniques. Recombinantly produced immunogenic peptides of the invention can be derived from a larger precursor protein, e.g., via enzymatic cleavage of enzyme cleavage sites inserted adjacent to the N- and/or C-terminus of the immunogenic peptide, followed by suitable purification.

In view of the limited length of the immunogenic peptides for use in the context of the invention, they can be prepared by chemical peptide synthesis, wherein peptides are prepared by coupling the different amino acids to each other. Chemical synthesis is particularly suitable for the inclusion of e.g. D-amino acids, amino acids with non-naturally occurring side chains or natural amino acids with modified side chains such as methylated cysteine. Chemical peptide synthesis methods are well described and peptides can be ordered from companies such as Applied Biosystems and other companies. Peptide synthesis can be performed as either solid phase peptide synthesis (SPPS) or contrary to solution phase peptide synthesis. The best-known SPPS methods are t-Boc and Fmoc solid phase chemistry which is amply known to the skilled person. In addition, peptides can be linked to each other to form longer peptides using a ligation strategy (chemoselective coupling of two unprotected peptide fragments) as originally described by Kent (Schnolzer & Kent (1992) Int. J. Pept. Protein Res. 40, 180-193) and reviewed for example in Tam et al. (2001) Biopolymers 60, 194-205. This provides the tremendous potential to achieve protein synthesis which is beyond the scope of SPPS. Many proteins with the size of 100-300 residues have been synthesised successfully by this method. Synthetic peptides have continued to play an ever-increasing crucial role in the research fields of biochemistry, pharmacology, neurobiology, enzymology and molecular biology because of the enormous advances in the SPPS.

The physical and chemical properties of an immunogenic peptide of interest (e.g. solubility, stability) is examined to determine whether the peptide is/would be suitable for use in therapeutic compositions. Typically this is optimised by adjusting the sequence of the peptide. Optionally, the peptide can be modified after synthesis (chemical modifications e.g. adding/deleting functional groups) using techniques known in the art.

In yet a further aspect, the present invention provides methods for generating soluble allofactor-specific cytotoxic T cells (Tregs or CD4+ regulatory T-cells) either *in vivo* or *in vitro (ex vivo).* In particular said T cells are cytotoxic towards any cell presenting a soluble allofactor antigen and are obtainable as a cell population. The invention extends to such (populations of) soluble allofactor-specific cytotoxic Tregs obtainable by the herein described methods.

In particular embodiments, methods are provided which comprise the isolation of peripheral blood cells, the stimulation of the cell population *in vitro* by contacting an immunogenic peptide according to the invention with the isolated peripheral blood cells, and the expansion of the stimulated cell population, more particularly in the presence of IL-2. The methods according to the invention have the advantage that higher numbers of Tregs are produced and that the Tregs can be generated which are specific for the soluble allofactor (by using a peptide comprising an antigen-specific epitope). Alternatively, soluble allofactor-specific cytotoxic T cells may be obtained by incubation in the presence of APCs presenting a soluble allofactor-specific immunogenic peptide according to the invention after transduction or transfection of the APCs with a genetic construct capable of driving expression of such immunogenic peptide. Such APCs may in fact themselves be administered to a subject in need to trigger in vivo in said subject the induction of the beneficial subset of cytotoxic CD4+ T-cells.

In an alternative embodiment, the Tregs can be generated *in vivo,* i.e. by the administration of an immunogenic peptide provided herein to a subject, and collection of the Tregs generated *in vivo.*

A further aspect of the invention relates to the use of the soluble allofactor-specific regulatory T cells obtainable by the above methods in the manufacture of a medicament for preventing or suppressing in a subject expected to receive, receiving or having received soluble allofactor the immune response to said soluble allofactor. For any of the above-described uses of the immunogenic peptides of the invention, said peptides can be replaced by said soluble allofactor-specific Tregs. Both the use of allogeneic and autogeneic cells is envisaged. Any method comprising the administration of said soluble allofactor-specific Tregs to a subject in need (i.e., for preventing or suppressing immune response(s) to a soluble allofactor) is also known as adoptive cell therapy. Such therapy is of particular interest in case of treating acute soluble allofactor-specific immune reactions and relapses of such reactions. Tregs are crucial in immunoregulation and have great therapeutic potential. The efficacy of Treg-based immunotherapy depends on the Ag specificity of the regulatory T cells. Moreover, the use of Ag-specific Treg as opposed to polyclonal expanded Treg reduces the total number of Treg necessary for therapy.

Yet a further aspect of the present invention relates to nucleic acid sequences encoding the immunogenic peptides described for use in the context of the present invention and methods for their use, e.g., for recombinant expression or in gene therapy. In particular, said nucleic acid sequences are capable of expressing the immunogenic peptides of the invention. The immunogenic peptides of the invention may indeed be administered to a subject in need by using any suitable gene therapy method. In any use or method of the invention for the treatment and/or suppression of immune response(s) to a soluble allofactor, immunisation with an immunogenic peptide of the invention may be combined with adoptive cell transfer of (a population of) Tregs specific for said immunogenic peptide and/or with gene therapy. When combined, said immunisation, adoptive cell transfer and gene therapy can be used concurrently, or sequentially in any possible combination.

In gene therapy, recombinant nucleic acid molecules encoding the immunogenic peptides can be used as naked DNA or in liposomes or other lipid systems for delivery to target cells. Other methods for the direct transfer of plasmid DNA into cells are well known to those skilled in the art for use in human gene therapy and involve targeting the DNA to receptors on cells by complexing the plasmid DNA to proteins. In its simplest form, gene transfer can be performed by simply injecting minute amounts of DNA into the nucleus of a cell, through a process of microinjection. Once recombinant genes are introduced into a cell, they can be recognised by the cells normal mechanisms for transcription and translation, and a gene product will be expressed. Other methods have also been attempted for introducing DNA into larger numbers of cells. These methods include: transfection, wherein DNA is precipitated with calcium phosphate and taken into cells by pinocytosis; electroporation, wherein cells are exposed to large voltage pulses to introduce holes into the membrane); lipofection/liposome fusion, wherein DNA is packed into lipophilic vesicles which fuse with a target cell; and particle bombardment using DNA bound to small projectiles. Another method for introducing DNA into cells is to couple the DNA to chemically modified proteins. Adenovirus proteins are capable of destabilising endosomes and enhancing the uptake of DNA into cells. Mixing adenovirus to solutions containing DNA complexes, or the binding of DNA to polylysine covalently attached to adenovirus using protein crosslinking agents substantially improves the uptake and expression of the recombinant gene. Adeno-associated virus vectors may also be used for gene delivery into vascular cells. As used herein, "gene transfer" means the process of introducing a foreign nucleic acid molecule into a cell, which is commonly performed to enable the expression of a particular product encoded by the gene. The said product may include a protein, polypeptide, anti-sense DNA or RNA, or enzymatically active RNA. Gene transfer can be performed in cultured cells or by direct administration into mammals. In another embodiment, a vector comprising a nucleic acid molecule sequence encoding an immunogenic peptide according to the invention is provided. In particular embodiments, the vector is generated such that the nucleic acid molecule sequence is expressed only in a specific tissue. Methods of achieving tissue-specific gene expression are well known in the art, e.g., by placing the sequence encoding an immunogenic peptide of the invention under control of a promoter, which directs expression of the peptide specifically in one or more tissue(s) or organ(s). Expression vectors derived from viruses such as retroviruses, vaccinia virus, adenovirus, adeno-associated virus, herpes viruses, RNA viruses or bovine papilloma virus, may be used for delivery of nucleotide sequences (e.g., cDNA) encoding peptides, homologues or derivatives thereof according to the invention into the targeted tissues or cell population. Methods which are well known to those skilled in the art can be used to construct recombinant viral vectors containing such coding sequences. Alternatively, engineered cells containing a nucleic acid molecule coding for an immunogenic peptide according to the invention may be used in gene therapy.

Where the administration of one or more peptides according to the invention is ensured through gene transfer (i.e. the administration of a nucleic acid which ensures expression of peptides according to the invention in vivo upon administration), the appropriate dosage of the nucleic acid can be determined based on the amount of peptide expressed as a result of the introduced nucleic acid.

A further aspect of the invention envisages medicaments which are usually, but not necessarily, a (pharmaceutical) formulations comprising as active ingredient at least one of the immunogenic peptides of the invention, a (population of) Tregs specific for said immunogenic peptide or a gene therapeutic vector capable of expressing said immunogenic peptide. Apart from the active ingredient(s), such formulation will comprise at least one of a (pharmaceutically acceptable) diluent, carrier or adjuvant. Typically, pharmaceutically acceptable compounds (such as diluents, carriers and adjuvants) can be found in, e.g., a Pharmacopeia handbook (e.g. US-, European- or International Pharmacopeia). The medicament or pharmaceutical composition of the invention normally comprises a (prophylactically or therapeutically) effective amount of the active ingredient(s) wherein the effectiveness is relative to the condition or disorder to be prevented or treated. In particular, the pharmaceutical compositions of the invention are vaccines for prophylactic or therapeutic application.

The medicament or pharmaceutical composition of the invention may need to be administered to a subject in need as part of a prophylactic or therapeutic regimen comprising multiple administrations of said medicament or composition. Said multiple administrations usual occur sequentially and the time-interval between two administrations can vary and will be adjusted to the nature of the active ingredient and the nature of the condition to be prevented or treated. The amount of active ingredient given to a subject in need in a single administration can also vary and will depend on factors such as the physical status of the subject (e.g., weight, age), the status of the condition to be prevented or treated, and the experience of the treating doctor, physician or nurse.

The term "diluents" refers for instance to physiological saline solutions. The term "adjuvant" usually refers to a pharmacological or immunological agent that modifies (preferably increases) the effect of other agents (e.g., drugs, vaccines) while having few if any direct effects when given by themselves. As one example of an adjuvant aluminium hydroxide (alum) is given, to which an immunogenic peptide of the invention can be adsorbed. Further, many other adjuvants are known in the art and can be used provided they facilitate peptide presentation in MHC-class II presentation and T cell activation. The term "pharmaceutically acceptable carrier" means any material or substance with which the active ingredient is formulated in order to facilitate its application or dissemination to the locus to be treated, for instance by dissolving, dispersing or diffusing the said composition, and/or to facilitate its storage, transport or handling without impairing its effectiveness. They include any and all solvents, dispersion media, coatings, antibacterial and antifungal agents (for example phenol, sorbic acid, chlorobutanol), isotonic agents (such as sugars or sodium chloride) and the like. Additional ingredients may be included in order to control the duration of action of the active ingredient in the composition. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, suspensions, ointments, creams, tablets, pellets or powders. Suitable pharmaceutical carriers for use in said pharmaceutical compositions and their formulation are well known to those skilled in the art, and there is no particular restriction to their selection within the present invention. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-steps procedure, with the selected carrier material and, where appropriate, the other additives such as surface-active agents. They may also be prepared by micronisation, for instance in view to obtain them in the form of microspheres usually having a diameter of about 1 to 10 µm, namely for the manufacture of microcapsules for controlled or sustained release of the active ingredients.

Immunogenic peptides, homologues or derivatives thereof according to the invention (and their physiologically acceptable salts or pharmaceutical compositions all included in the term "active ingredients") may be administered by any route appropriate to the condition to be prevented or treated and appropriate for the compounds, here the immunogenic proteins to be administered. Possible routes include regional, systemic, oral (solid form or inhalation), rectal, nasal, topical (including ocular, buccal and sublingual), vaginal and parenteral (including subcutaneous, intramuscular, intravenous, intradermal, intraarterial, intrathecal and epidural). The preferred route of administration may vary with for example the condition of the recipient or with the condition to be prevented or treated.

The formulations may conveniently be presented in unit dosage form and may be prepared by any of the methods well known in the art of pharmacy. Formulations of the present invention suitable for oral administration may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste. A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

A further aspect of the invention relates to isolated immunogenic peptides comprising a T-cell epitope from a soluble allofactor and, adjacent to said T-cell epitope or separated from said T-cell epitope by a linker, a C-(X)2-[CST] or [CST]-(X)2-C motif. In particular embodiments immunogenic peptides are provided which are derived from soluble allofactors which do not comprise in their natural sequence an epitope and a redox motif within 11 amino acids Nor C-terminally adjacent to said epitope. In particular embodiments the allofactor is a therapeutic replacement agent.

An alternative strategy to treat alloimmunisation responses to soluble factors consists of targeting memory B cells secreting antibodies to said soluble factor. This is due to the property of memory B cells to express surface BCR (B-cell receptor) containing the variable chains of an antibody identical to the one they secrete after activation (a memory B cells produce an antibody only after seeing the antigen and being activated by corresponding CD4+ T cells directed towards epitopes derived from either the antigen or from BCR idiotype). An idiotype is made of the ensemble of antigenic determinants carried by variable part of antibodies. Hence, said BCR and the secreted antibody share idiotypic determinants. During uptake of polypeptides or proteins by B cells, parts of the BCR are processed together with the antigen and are presented by MHC class II determinants. Therefore, memory B cells also present CD4+ T cell epitopes derived from their own BCR. CD4+ T cells directed towards such BCR-derived epitopes can activate the corresponding B cells. As T-cell epitopes modified by attaching a redox motif thereto induce CD4+ T-cells to acquire the property of inducing apoptosis in APCs presenting said T-cell epitope (natural or modified), memory B-cell BCR T-cell epitopes modified by attaching a redox motif thereto are capable of inducing CD4+ T-cells that can drive said memory B-cells into apoptosis. Thus, the memory B cells towards soluble allofactors can be eliminated by using an allofactor-specific memory B-cell BCR T-cell epitope modified by attaching a redox motif thereto. This approach is of special interest for situations in which the response towards the soluble allofactor is oligocional which is often the case. This alternative strategy can obviously also be combined with the strategy described above which is based on using T-cell epitopes derived from the allofactors themselves. Furthermore, this alternative strategy could also be employed in a preventive setting in situations wherein memory B cells are present without soluble antibodies being detectable. In such cases, the above idiotype-orientated strategy would be preventive in so far as the elimination of memory B cells would prevent the production of antibodies when antigen is around. In particular cases the above strategy involving T-cell epitopes of BCR or idiotypes thereof may be extended to naïve B cells. Indeed, in cases of antibodies (and therefore BCR) being in the germline configuration, namely with exactly the same sequence at the level of variable parts as naïve B cells produced by random gene rearrangement in the bone marrow. Immunisation with a idiotype-derived modified T-cell epitope (to induce cytotoxic CD4+ regulatory T-cells specific to the idiotype T-cell epitope) would prevent the selection of such B cells. Examples of relevant antibodies in germline configuration are antibodies binding to the C2 domain of Factor VIII, thereby inhibiting the function of FVIII.

Hence, a further aspect of the invention relates to the use of at least one isolated immunogenic peptide comprising (i) a T-cell epitope derived from an anti-allofactor antibody idiotype and (ii) a C-(X)2-[CST] or [CST]-(X)2-C motif, for the manufacture of a medicament for (substantially) eliminating allofactor-specific B cells in a subject having received said allofactor.

The invention likewise relates to the use of at least one isolated immunogenic peptide comprising (i) a T-cell epitope derived from an anti-allofactor antibody idiotype and (ii) a C-(X)2-[CST] or [CST]-(X)2-C motif, for the manufacture of a medicament for preventing activation of CD4+ effector T-cells capable of activating allofactor-specific B cells in a subject having received said allofactor.

The invention further relates to the use of at least one isolated immunogenic peptide comprising (i) a T-cell epitope derived from an anti-allofactor antibody idiotype and (ii) a [CST]-(X)2-[CST] motif, more particularly a C-(X)2-[CST] or [CST]-(X)2-C motif for the manufacture of a medicament for inducing in a recipient CD4+ regulatory T cells which are cytotoxic to allofactor-specific B cells in a subject having received said allofactor.

In any of these aspects, the B cells are memory B cells or naïve B cells.

The present invention will now be illustrated by means of the following examples, which are provided without any limiting intention. Furthermore, all references described herein are explicitly included herein by reference.

### EXAMPLES

### EXAMPLE 1. Induction into apoptosis of splenic B cells of naïve mice presenting a peptide in MHC class II determinants

It was determined whether naïve B cells presenting a class II restricted T cell epitope derived from a BCR idiotype could be deleted by recognition and activation of T cells elicited to a specific anti-factor VIII antibody carrying the same idiotype. Thus, C57BI/6 mice were immunised 3 times at a fortnight interval with Fab fragments of antibody BO2C11, a human monoclonal antibody to the C2 domain of factor VIII (Jacquemin et al. (1998) Blood 92, 496-501). Ten days after the last immunisation, the mice were sacrificed and CD4+ T cells prepared from their spleen by magnetic bead sorting.

CD4+ T cells were then expanded in vitro by presentation of/contacting with a peptide identified using the above-referenced algorithms as carrying a T cell epitope. This T cell epitope is derived from the complementarity-determining region (CDR) 3 of the VH region of the BO2C11 antibody. This epitope is of sequence: YCAVPDDPDA (SEQ ID NO:1).

In parallel experiments, CD4+ T cells were stimulated with a modified version of the T cell epitope, the modification consisting of the addition of a consensus sequence with thioreductase activity ("redox motif"). The modified epitope is of sequence: CHGCYCAVPDPDA (SEQ ID NO:2; sequence of redox motif underlined).

Naïve B cells were loaded by incubation with a peptide of either SEQ ID NO:1 or SEQ ID NO:2 and washed. Each of these two B cell cultures was then mixed with T cells stimulated with either the peptide of SEQ ID NO:1 or the peptide of SEQ ID NO:2. A control population of naïve B cells was cultured in parallel to evaluate the spontaneous loss of B cells.

T cells expanded with peptide of SEQ ID NO:1, namely the T cell epitope in each natural conformation showed no or only little influence on the survival of control B cells over an incubation period of 18 h (data not shown). By contrast, B cells loaded with either peptide of SEQ ID NO:1 or peptide of SEQ ID NO:2 were induced into apoptosis (see Figure 1) by T cells expanded with peptide of SEQ ID NO:2.

It can therefore be concluded that the addition of a redox motif to the T-cell epitope (natural epitope of SEQ ID NO:1 to modified epitope of SEQ ID NO:2) was sufficient to alter the properties of CD4+ T cells elicited to the antibody idiotype in such a way as to induce apoptosis of target B cells. Besides, the CD4+ T cells with cytotoxic activity could be activated by recognition of the natural T cell epitope (peptide of sequence 1).

A phenotypic evaluation of cytotoxic T cells induced in the present experiment indicated that they shared markers of regulatory T cells, such as high expression of CD25 at rest, with high CTLA-4 and GITR. However, no Foxp3 transcription repressor was detected. Factors involved in the induction of apoptosis were readily expressed, including Fas and FasL, and granzymes.

### EXAMPLE 2. Induction into apoptosis of human B cells specific for factor VIII by CD4+ T cells specific to a factor VIII epitope presented into MHC class II molecules

Human lymphoblastoid B cell lines were obtained from the peripheral blood of a patient affected by a mild form of haemophilia and producing antibodies neutralising factor VIII function. A specific cell line, LE2E9 (referred to hereinafter as 2E9) produced an antibody to the carboxyterminal end of the factor VIII C1 domain (Jacquemin et al. (2000), Blood 95, 156-163). The 2E9 cell line was shown to present factor VIII derived peptides within the context of MHC class II molecule, which resulted in specific CD4+ T cell activation. Such CD4+ T cells were cloned from the peripheral blood of the same patient. The peptide recognised by such T cell clones was mapped and is of sequence: IIARY-IRLHPTHYSIRST (SEQ ID NO:3), which corresponds to amino acids 2144 to 2161 of the C1 domain and in which I in position 2149 corresponds to the first MHC anchoring residue (P1).

This peptide is modified by replacing amino acids 2144 to 2148 by a sequence CGHCGG, encoding a consensus sequence with thioreductase activity ("redox motif"). The modified peptide is of sequence: CGHCGG-IRLHPTHYSIR (SEQ ID NO:4; wherein the redox motif is underlined).

A specific T cell clone (Jacquemin et al. (2003), Blood 101, 1351-1358) is cultured on APC (dendritic cells) presenting either peptide of SEQ ID NO:3 or of SEQ ID NO:4. After a period of rest, cells are added to cultures of the 2E9 B cell line loaded over a period of 4 hours with either peptide of SEQ ID NO:3 or of SEQ ID NO:4 and then washed.

The effect of T cell clones expanded with the natural epitope of SEQ ID NO:3 or the modified peptide of SEQ ID NO:4 on induction of apoptosis of the 2E9 lymphoblastoid cells presenting either the natural epitope of SEQ ID NO:3 or its modified counterpart of SEQ ID NO:4 is compared.

### EXAMPLE 3. Induction into apoptosis of human dendritic cells specific for factor VIII by CD4+ T cells specific to a factor VIII epitope

To determine whether a primary immune response to factor VIII could be prevented using modified epitopes extended with a redox motif, an experiment similar to that described in Example 2 is carried out using dendritic cells instead of a B cell line. Thus, human dendritic cells are prepared from peripheral blood monocytes by culturing these in the presence of GM-CSF and IL-4. Full maturation is then obtained by addition of TNF-alpha, according to published methods.

Dendritic cells are loaded by incubation with peptides of SEQ ID NO:3 or SEQ ID NO:4 comprising a T cell epitope of FVIII (see Example 2). After washing, dendritic cells are incubated with the factor VIII-specific T cell clone pre-activated with either peptide of SEQ ID NO:3 or SEQ ID NO:4.

These experiments demonstrate the capability of T-cell epitopes modified by the addition of a redox motif to prevent a primary response to an alloantigen by the induction of apoptosis of APC presenting a specific T cell epitope.

### EXAMPLE 4. Suppression of a secondary immune response to an alloantigen by eliciting cytotoxic regulatory T cells to either factor VIII or to BCR-derived idiotypes

To determine whether cytotoxic regulatory T cells can suppress a secondary immune response, we take advantage of a transgenic mouse strain expressing a B cell receptor (BCR) to human factor VIII. Transgenic B cells are isolated from the spleen of such mice by sorting out with magnetic beads.

The isolated transgenic B cells are incubated with factor VIII and washed. The cells are then co-cultured with polyclonal T cells obtained from the spleen of a mouse immunised with human factor VIII. Such splenocytes contain CD4+ T cells specific to human factor VIII, which are purified by sorting on magnetic beads. T cells are then cultured with transgenic B cells presenting factor VIII and finally cloned by limiting dilution. Clones recognising the peptide of SEQ ID NO:3 are expanded.

Mouse T cell clones to factor VIII are activated by incubation with APC presenting either peptide of SEQ ID NO:3 (natural T cell epitope of factor VIII) or peptide of SEQ ID NO:4 (modified factor VIII epitope). After a resting period of 10 days, each of these pre-activated T cell clones are incubated for 18 h with transgenic B cells presenting factor VIII. The effect of the differently activated T-cells on the transgenic B cells is assessed.

As the transgenic B cells also express T cell epitopes derived from the BCR, we use the same system to determine whether an ongoing immune response to a given alloantigen can be suppressed by generating cytotoxic regulatory T cells to idiotypic determinants.

As the transgenic BCR are derived from human anti-factor VIII antibody BO2C11, the transgenic B cell line presents the same idiotypic determinants as those described in Example 1 and therefore the T cell clones used in Example 1 can be used.

Transgenic B cells are incubated with factor VIII and washed. The cells are then co-cultured with the T cell clones of Example 1 pre-activated with peptide of SEQ ID NO:1 (natural idiotypic determinant) or peptide of SEQ ID NO:2 (modified idiotypic determinant). The effect of the different activated T-cells on the transgenic B cells is assessed.

### EXAMPLE 5. Induction into apoptosis of factor VIII-specific effector CD4+ T cells by bystander suppression induced by in vivo elicited Factor VIII-specific cytolytic CD4+ T cells

Polyclonal effector CD4+ T cells were obtained from the spleen of factor VIII KO mice immunised with 2 IU of recombinant human FVIII (CD4(F8-)) and purified using anti-CD4 magnetic beads. The cells were then labelled with CFSE.

Induction of apoptosis as measured by of CFSE-labelled cells was then measured by Annexin V and 7-AAD staining after incubating such cells with APC (spleen B cells) loaded with 5 µg/ml huFVIII and 1 µM of peptide of SEQ ID NO:5 (CGHCGGFTNMFATWSPSK, corresponding to the 2196-2207 amino acid sequence of the C2 domain of human factor VIII, modified by addition of a thioreductase motif (underlined) separated by 2 glycines from the Factor VIII T-cell epitope). Apoptosis was measured after 72 h culture at 37°C.

When CFSE-labelled CD4(F8-) cells were co-cultured with CD4+ T cells obtained from mice immunised with a synthetic peptide of SEQ ID NO:5 (cytolytic CD4+ T cells), significant apoptosis was induced. Figure 2 shows that two independent preparations of cytolytic CD4+ T cells produced 6.6% and 8.4% of apoptosis, respectively ("CD4(F8+) pool1" and "CD4(F8+)pool2" in Figure 2). When CFSE-labelled CD4(F8-) cells were co-cultured in the presence of a double number of unlabeled CD4(F8-) cells, no apoptosis was induced ("CD4(F8-)" caption in Figure 2). Baseline mortality was subtracted from percentages of cell death. These results indicate that immune responses to soluble allofactors relying on effector CD4+ T-cells can be eliminated by cytolytic CD4+ T-cells elicited using an allofactor-derived T-cell epitope modified according to the invention.

## Claims

1. An isolated immunogenic peptide comprising (i) a T-cell epitope derived from a soluble allofactor and (ii) a C-(X)2-[CST] or [CST]-(X)2-C motif, wherein said motif is immediately adjacent to said T-cell epitope, or is separated from said T-cell epitope by a linker of at most 7 amino acids,
for use in preventing or suppressing, in a subject expected to receive, receiving or having received said soluble allofactor, an immune response to said soluble allofactor.

2. The peptide according to claim 1, for use in the method of claim 1, wherein said soluble allofactor is used in replacement therapy.

3. The peptide according to claim 1 or 2, for use in the method of claim 1, wherein said soluble allofactor is selected from the group consisting of a coagulation factor, a fibrinolytic factor, a hormone, a cytokine, a growth factor and an antibody used for therapeutic purpose.

4. The peptide according to any one of claim 1 to 3, for use in the method of claim 1, wherein said motif does not naturally occur within a region of 11 amino acids N- or C-terminally adjacent to the T-cell epitope in said soluble allofactor.

5. The peptide according to any one of claims 1 to 4, for use in the method of claim 1, wherein said motif is positioned N-terminally of the T-cell epitope.

6. The peptide according to any one of claims 1 to 5, for use in the method of claim 1, wherein :
at least one X in said motif is Gly, Ala, Ser or Thr, or wherein at least one X in said motif is His or Pro, or wherein at least one C in said motif is methylated.

7. A method for obtaining a population of soluble allofactor-specific regulatory T cells with cytotoxic properties, the method comprising the steps of:
providing peripheral blood cells;
contacting said cells in vitro with an immunogenic peptide comprising (i) a T-cell epitope derived from a soluble allofactor and (ii) a C-(X)2-[CST] or [CST]-(X)2-C motif, wherein said motif is immediately adjacent to said T-cell epitope, or is separated from said T-cell epitope by a linker of at most 7 amino acids; and
expanding said cells in the presence of IL-2.

8. A method for obtaining a population of soluble allofactor-specific regulatory T cells with cytotoxic properties, the method comprising the steps of:
providing an immunogenic peptide comprising (i) a T-cell epitope derived from a soluble allofactor and (ii) a C-(X)2-[CST] or [CST]-(X)2-C motif, wherein said motif is immediately adjacent to said T-cell epitope, or is separated from said T-cell epitope by a linker of at most 7 amino acids;
administering said immunogenic peptide to a subject; and
obtaining said population of soluble allofactor-specific regulatory T cells from said subject.

9. A population of soluble allofactor-specific regulatory T cells with cytotoxic properties obtainable by the method according to claim 7 or 8.

10. A population of soluble allofactor-specific regulatory T cells according to claim 9 for use in preventing or suppressing, in a subject to receive, receiving or having received said soluble allofactor, the immune responses to said soluble allofactor.

11. An isolated immunogenic peptide comprising a T-cell epitope from a soluble allofactor and, immediately adjacent to said T-cell epitope or separated from said T-cell epitope by a linker of at most 7 amino acids, a C-(X)2-[CST] or [CST]-(X)2-C motif.

12. An isolated immunogenic peptide comprising (i) a T-cell epitope derived from an anti-allofactor antibody idiotype and immediately adjacent to said T-cell epitope or separated from said T-cell epitope by a linker of at most 7 amino acids (ii) a C-(X)2-[CST] or [CST]-(X)2-C motif, for use in eliminating an allofactor-specific B cells in a subject having received said allofactor.

13. A method of producing an immunogenic peptide with a length of between 12 and 50 amino acids, capable of eliciting CD4+ T cell activity, the method comprising the steps of:
a) identifying in a sequence of a soluble allofactor an MHC class II T cell epitope,
b) producing a peptide comprising
- the sequence of the epitope identified in step a) and
- a [CST]-X(2)-C or C-X(2)-[CST] redox motif sequence, such that said T cell epitope sequence and said redox motif sequence are immediately adjacent to each other or separated from each other by at most 7 amino acids.

14. The method according to claim 13, wherein said soluble allofactor does not have a [CST]-X(2)-C or C-X(2)-[CST] sequence within a region of 11 amino acids, N-terminally or C-terminally of said T cell epitope in said soluble allofactor.


SEQUENCE LISTING

<110>  Life Sciences Research Partners VZW
       Saint-Remy, Jean-Marie

<120>  Strategies to prevent and/or treat immune responses to soluble
       allofactors

<130>  T5212-PCT (043)

<150>  EP 08447010.3
<151>  2008-02-14

<150>  US 61/035,800
<151>  2008-03-12

<160>  16

<170>  PatentIn version 3.3

<210>  1
<211>  10
<212>  PRT
<213>  Artificial Sequence

<220>
<223>  T-cell epitope of CDR3 of anti-factor VIII antibody BO2C11

<400>  1

Tyr Cys Ala Val Pro Asp Asp Pro Asp Ala
1               5                   10


<210>  2
<211>  13
<212>  PRT
<213>  Artificial Sequence

<220>
<223>  modified T-cell epitope of CDR3 of anti-factor VIII antibody
       BO2C11


<220>
<221>  MISC_FEATURE
<222>  (1)..(4)
<223>  thioreductase motif

<400>  2

Cys His Gly Cys Tyr Cys Ala Val Pro Asp Pro Asp Ala
1               5                   10


<210>  3
<211>  18
<212>  PRT
<213>  Artificial Sequence

<220>

<223>  amino acids 2144-2161 of human factor VIII

<400>  3

Ile Ile Ala Arg Tyr Ile Arg Leu His Pro Thr His Tyr Ser Ile Arg
1               5                   10                  15

Ser Thr

<210>  4
<211>  17
<212>  PRT
<213>  Artificial Sequence

<220>
<223>  modified T-cell epitope of human factor VIII

<220>
<221>  MISC_FEATURE
<222>  (1)..(4)
<223>  thioreductase motif

<220>
<221>  MISC_FEATURE
<222>  (5)..(6)
<223>  Gly-Gly linker

<400>  4

Cys Gly His Cys Gly Gly Ile Arg Leu His Pro Thr His Tyr Ser Ile
1               5                   10                  15

Arg

<210>  5
<211>  18
<212>  PRT
<213>  Artificial Sequence

<220>
<223>  modified human factor VIII T-cell epitope

<220>
<221>  MISC_FEATURE
<222>  (1)..(4)
<223>  thioreductase motif

<220>
<221>  MISC_FEATURE
<222>  (5)..(6)
<223>  Gly-Gly-linker

<400>  5

Cys Gly His Cys Gly Gly Phe Thr Asn Met Phe Ala Thr Trp Ser Pro
1               5                   10                  15

Ser Lys

<210>  6
<211>  6
<212>  PRT
<213>  Artificial Sequence

<220>
<223>  general sequence of peptide of the invention

<220>
<221>  MISC_FEATURE
<222>  (2)..(3)
<223>  Xaa at positions 2 and 3 denote any amino acid

<220>
<221>  MISC_FEATURE
<222>  (6)..(6)
<223>  Xaa denotes the sequence of any T-cell epitope

<400>  6

Cys Xaa Xaa Cys Gly Xaa
1               5

<210>  7
<211>  7
<212>  PRT
<213>  Artificial Sequence

<220>
<223>  general sequence of peptide of the invention

<220>
<221>  MISC_FEATURE
<222>  (2)..(3)
<223>  Xaa at positions 2 and 3 denote any amino acid

<220>
<221>  MISC_FEATURE
<222>  (7)..(7)
<223>  Xaa denotes sequence of any T-cell epitope

<400>  7

Cys Xaa Xaa Cys Gly Gly Xaa
1               5

<210>  8
<211>  8

<212>  PRT
<213>  Artificial Sequence

<220>
<223>  general sequence of peptide of the invention


<220>
<221>  MISC_FEATURE
<222>  (2)..(3)
<223>  Xaa at positions 2 and 3 denote any amino acid

<220>
<221>  MISC_FEATURE
<222>  (8)..(8)
<223>  Xaa denotes sequence of any T-cell epitope

<400>  8

Cys Xaa Xaa Cys Ser Ser Ser Xaa
1               5


<210>  9
<211>  9
<212>  PRT
<213>  Artificial Sequence

<220>
<223>  general sequence of peptide of the invention


<220>
<221>  MISC_FEATURE
<222>  (2)..(3)
<223>  Xaa at positions 2 and 3 denote any amino acid

<220>
<221>  MISC_FEATURE
<222>  (9)..(9)
<223>  Xaa denotes sequence of any T-cell epitope

<400>  9

Cys Xaa Xaa Cys Ser Gly Ser Gly Xaa
1               5


<210>  10
<211>  14
<212>  PRT
<213>  Artificial Sequence

<220>
<223>  thioreductase motif repeat


<220>
<221>  MISC_FEATURE
<222>  (1)..(14)
<223>  Xaa at positions 2, 3, 5, 7, 8, 10, 12, and 13 denote any amino

acid

<400>  10

Cys Xaa Xaa Cys Xaa Cys Xaa Xaa Cys Xaa Cys Xaa Xaa Cys
1               5                   10


<210>  11
<211>  12
<212>  PRT
<213>  Artificial Sequence

<220>
<223>  thioreductase motif repeat


<220>
<221>  MISC_FEATURE
<222>  (1)..(12)
<223>  Xaa at positions 2, 3, 6, 7, 10, and 11 denote any amino acid

<400>  11

Cys Xaa Xaa Cys Cys Xaa Xaa Cys Cys Xaa Xaa Cys
1               5                   10


<210>  12
<211>  10
<212>  PRT
<213>  Artificial Sequence

<220>
<223>  thioreductase motif repeat


<220>
<221>  MISC_FEATURE
<222>  (1)..(10)
<223>  Xaa at positions 2, 3, 5, 6, 8, and 9 denote any amino acid

<400>  12

Cys Xaa Xaa Cys Xaa Xaa Cys Xaa Xaa Cys
1               5                   10


<210>  13
<211>  10
<212>  PRT
<213>  Artificial Sequence

<220>
<223>  thioreductase motif repeat


<220>
<221>  MISC_FEATURE
<222>  (1)..(10)
<223>  Xaa at positions 2, 5, and 8 denote any amino acid

<400>  13

Cys Xaa Cys Cys Xaa Cys Cys Xaa Cys Cys
1               5                   10



<210>  14
<211>  6
<212>  PRT
<213>  Artificial Sequence

<220>
<223>  late endosome targeting signal



<220>
<221>  MISC_FEATURE
<222>  (1)..(1)
<223>  Xaa denotes aspartate (D or Asp) or glutamate (E or Glu)

<220>
<221>  MISC_FEATURE
<222>  (2)..(4)
<223>  Xaa at positions 2, 3 and 4 denote any amino acid

<220>
<221>  MISC_FEATURE
<222>  (6)..(6)
<223>  Xaa denotes leucine (L or Leu) or isoleucine (I or Ile)

<400>  14

Xaa Xaa Xaa Xaa Leu Xaa
1               5



<210>  15
<211>  5
<212>  PRT
<213>  Artificial Sequence

<220>
<223>  late endosome targeting signal



<220>
<221>  MISC_FEATURE
<222>  (2)..(3)
<223>  Xaa at positions 2 and 3 denote any amino acid

<400>  15

Asp Xaa Xaa Leu Leu
1               5



<210>  16
<211>  6
<212>  PRT
<213>  Artificial Sequence

<220>
<223>  late endosome targeting signal

<220>
<221>  MISC_FEATURE
<222>  (2)..(5)
<223>  Xaa at positions 2, 3 and 4 denote any amino acid

<400>  16

Asp Xaa Xaa Xaa Leu Leu
1               5